# EUROPEAN PATENT SPECIFICATION

(11) **EP 1 948 224 B1**
(45) Date of publication and mention of the grant of the patent: **12.03.2014**
(21) Application number: 06813152.3
(22) Date of filing: 08.11.2006
(51) Int. Cl.: A61K 38/55, A61K 31/401, A61K 9/16, A61K 9/19, A61K 9/20, C07D 209/42, A61P 9/12

(54) **STABLE FORMULATION OF AMORPHOUS PERINDOPRIL SALTS, A PROCESS FOR THEIR PREPARATION, ESPECIALLY INDUSTRIAL PREPARATION, AND THEIR USE IN THE THERAPY OF HYPERTENSION**
STABILE FORMULIERUNG VON AMORPHEM PERINDOPRIL-SALZEN, VERFAHREN ZU IHRER HERSTELLUNG, INSBESONDERE INDUSTRIELLEN HERSTELLUNG UND IHRE VERWENDUNG BEI DER BEHANDLUNG VON HYPERTONIE
FORMULATION STABLE DE SELS AMORPHES DE PERINDOPRIL, PROCÉDÉ POUR LA PRÉPARATION, EN PARTICULIER POUR LA PRÉPARATION INDUSTRIELLE DE CEUX-CI ET UTILISATION DE CEUX-CI DANS LA THÉRAPIE DE L'HYPERTENSION

(30) Priority: 17.11.2005 SI 200500314; 31.07.2006 SI 200600177
(43) Date of publication of application: 30.07.2008
(73) Proprietor: Silverstone Pharma Est., 9490 Vaduz (LI)
(72) Inventor: RUCMAN, Rudolf, 1211 Smartno Pri Ljubljani (SI); ZUPET, Pavel, 8000 Novo Mesto (SI)
(74) Representative: Ros, Zlata
(86) International application number: PCT/SI2006/000034
(87) International publication number: WO 2007/058634

(56) References cited:
- EP-A- 1 334 716
- WO-A-2005/068425
- WO-A-2005/068490
- WO-A-2005/094793
- WO-A2-03/075842
- US-A- 4 743 450
- US-A- 5 350 582
- US-A- 5 573 780
- COLACO C ET AL: "EXTRAORDINARY STABILITY OF ENZYMES DRIED IN TREHALOSE: SIMPLIFIED MOLECULAR BIOLOGY" BIO/TECHNOLOGY, NATURE PUBLISHING CO. NEW YORK, US, vol. 10, no. 9, 1 September 1992 (1992-09-01), pages 1007-1011, XP000568746 ISSN: 0733-222X cited in the application
- "British Pharmacopoeia", 1 January 2004 (2004-01-01)
- 'Organic Chemistry of Drug Degradatiopn',

## Description

### Technical Field

The present invention belongs to the field of pharmaceutical chemistry and relates to a stable formulation of an amorphous perindopril sodium salt of the formula I: 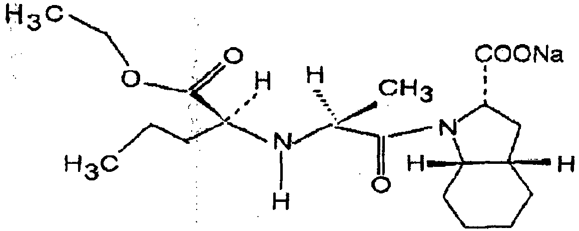 a process for its preparation, especially on industrial scale, and its use in treating hypertension.

### Technical Problem

A perindopril sodium salt is a very effective vasodilatator and antihypertensive agent. Because of its great pharmaceutical importance there was a need for a novel, especially industrially applicable process for the preparation of a stable formulation thereof.

### Prior Art

In known formulations the active component perindopril erbumine is in different polymorphic forms of the formula II:

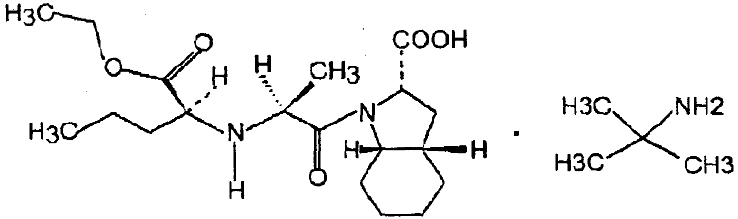

A formulation with perindopril erbumine in α-polymorphic form is disclosed in WO 01/87835 A1 to Servier. Perindopril erbumine in β-polymorphic form enters the formulation of a pharmaceutical form according to WO 01/87836 A1 to Adir & Co.

Perindopril erbumine in γ-polymorphic form disclosed in US 2003/0158121 A1 and in WO 01/83439 A1 to Adir & Co is less suitable for pharmaceutical formulations due to the manner of preparing the compound - crystallization by means of chloroform, a solvent that, even in traces, is not admissible in pharmaceutical formulations. Neither of said processes teaches special additives for enhancing the stability of pharmaceutical preparations. However, WO 03/075842 A2 to TEVA discloses stable formulations containing ACE inhibitors, especially moexipril with the addition of sodium hydrogen carbonate.

The decomposition of ACE inhibitors may lake place in several ways:
- by hydrolysis of the ester group, especially in a side chain,
- by water elimination and internal cyclization where diketopiperazines are formed.

From the patent literature several processes are known which deal with more stable formulations that should prevent these decomposition reactions, e.g. US 4,743,450.

US 5,573,780 and US 5,690,962 disclose stable formulations of enalapril and a process for the preparation thereof. SI 9111842 A and US 5,350,582 disclose stable formulations of enalapril with the addition of a stoichiometric amount of various basic additives, preferably alkali metal carbonates, which are added in solid form.

Magnesium carbonate as a means for stabilization of quinapril preparations is disclosed in US 4,473,450.

In DE 10 2004 019 845 A1 there is disclosed the manufacture of a preparation with perindopril erbumine, which preparation contains a crystallized α-form of perindopril erbumine admixed in solid state with sodium hydrogen carbonate for stabilizing. In this case the effect of stabilization is not perfect since it is only perceived at the contact of two solid surfaces. Besides, there is used the crystal α-form claimed in other patent documents, e.g. WO 01/87835.

WO 03/061691 A1 to Servier discloses pharmaceutical preparations of perindopril for oral application, which are dissolved already in the oral cavity and contain additives such as Starlac®, sodium stearyl fumarate and colloidal silicium dioxide. Stability data are not given, but in view of the application route and the short resorption, stability is only important for the durability of the preparation.

A simple pharmaceutical formulation without special additives for enhancing stability is disclosed in WO 2004/046172 (PCT/GB 2003/004981) and GB 2,395,195 A to Cipla where perindopril erbumine hydrates enter the formulation.

Perindopril sodium salt is disclosed in EP 0049658 and US 6,696,481, where the sodium salt is found unsuitable for pharmaceutical use and also not suitable for handling because, on contact with the atmosphere, it is immediately converted into an oil and degraded rapidly.

WO 2005/068490 of Lek describes inclusion complexes of perindopril erbumine with cyclodextrins.

WO 2005/094793 of Krka describes a preparation of a solid pharmaceutical form, wherein perindopril erbumine in a dry solid form is mixed with other solid ingredients.

### The Inventive Solution

A stable formulation of perindopril sodium salt in an amorphous form is prepared according to the present invention in such a way that perindopril erbumine is dissolved in water or an alcohol/water mixture in any ratio. To this solution an aqueous solution of sodium hydrogen carbonate in a ratio 1 mole per 1 mole of perindopril erbumine is added and the whole is blended. Then it is added by spraying to a homogenous mixture of inert ingredients for the preparation of a granulate, dried in a stream of warm air with a temperature over 40 °C, substances to facilitate tabletting are added, the whole is homogenized and the granulate is formulated.

As ingredients for preparing granulates standard inert substances such as e.g. microcrystalline cellulose, anhydrous lactose or its monohydrate and corn starch are used. More hydrophobic substances such as magnesium stearate and talc, which facilitate tabletting, are added most preferably in the second phase of granulate preparation.

When tablets containing perindopril erbumine stand, especially at higher temperatures and increased air humidity, several decomposition products may be formed, two of them being very important: diketopiperazine (abbr. DKP or impurity F according to pharmacopoeia), which is chemically ethyl(2S)-2-[(3S,5aS,9aS,10aS)-3-methyl-1,4-dioxodecahydropyrazino[1,2a]indol-2(1H)yl]pentanoate, and a hydrolysis product (impurity B), which is chemically (2S,3aS,7aS)-1-[(2S)-2-[[(1S)-1-carboxybutyl]amino]propanoyl]octahydro- 1H-indole-2-carboxylic acid. Tert-butylamine in the molecule of perindopril erbumine is only weakly bonded. Due to its very low boiling point (about 44 °C) tert-butylamine is slowly released from molecule, which accelerates the formation of impurity F. Surprisingly, in the case of perindopril sodium salt of the present invention, the bond between sodium and perindopril is much stronger and therefore this decomposition is inhibited .

After drying at a higher temperature (over 40°C) the obtained sodium salt in amorphous form is surprisingly stable and also suitable for any pharmaceutical preparation.

Surprisingly, the mass after drying as well as after completed homogenization and in tablets does not contain a perindopril sodium salt in any known or patented crystal form.

The invention is illustrated by the following Example, which in no way represents a limitation thereof.

### Example 1

Composition for 1000 tablets each containing 4 mg of perindopril sodium salt:

| | |
|---|---|
| perindopril erbumine | 4.0 g |
| lactose, anhydrous | 60.0 g |
| microcrystalline cellulose | 18.0 g |
| corn starch | 5.0 g |
| magnesium stearate | 1.0 g |
| sodium hydrogen carbonate | 0.76 g |
| talc | 2.0 g |
| Total | 90.76 g |

### Granulate preparation:

Perindopril erbumine (4.0 g) was dissolved in demineralized water (25 ml) and the clear solution was diluted with ethanol (20 ml). A solution of sodium hydrogen carbonate (0.76 g) dissolved in water (15 ml) was added, it was well blended and rapidly filtered.

Lactose (60 g), microcrystalline cellulose (18 g), corn starch (5 g), magnesium stearate (1 g) and talc (2 g) were separately blended and this mixture was wetted with the solution prepared above.

The wetted mixture was dried at 50°C with warm air until the water assay in the product was under 1% (K. Fischer).

## Claims

1. Process for the preparation, especially the industrial preparation, of a stable formulation of an amorphous perindopril sodium salt of formula I: 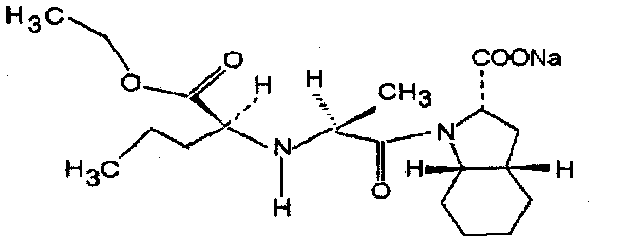 **characterized in that** perindopril erbumine of formula II: 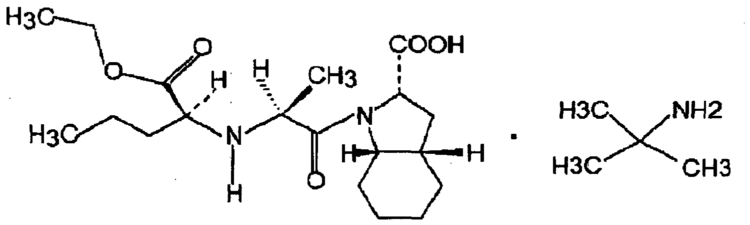 is dissolved in water or an alcohol/water mixture in any ratio, to this solution an aqueous solution of sodium hydrogen carbonate in a ratio 1 mole per 1 mole of perindopril erbumine is added and the whole is blended and added by spraying to a homogenous mixture of inert ingredients for the preparation of a granulate, dried in a stream of warm air with the temperature over 40 °C, substances to facilitate tabletting are added, it is homogenized and the granulate is formulated.

2. Process according to claim 1, **characterized in that** as inert ingredients for the preparation of the granulate lactose, lactose in the form of monohydrate, trehalose, corn starch, microcrystalline cellulose, talc and magnesium stearate are used.

3. Stable formulation of amorphous perindopril sodium salt granulate obtainable according to the process of claims 1-2.

4. Formulation according to claim 3, **characterized in that** it is in the form of a granulate, a tablet or a capsule.

5. Use of the formulation according to claims 3-4 for the preparation of a medicine having an antihypertensive and a vasodilatatory action.

## Patentansprüche

1. Verfahren zur Herstellung, insbesondere technischen Herstellung, einer stabilen Formulierung eines amorphen Perindopril-Natriumsalzes der Formel I: 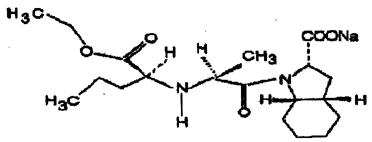 **dadurch gekennzeichnet, dass** man Perindopril-Erbumin der Formel II: 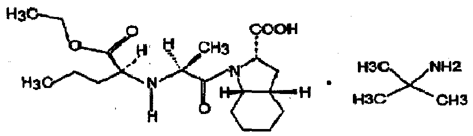 in Wasser oder einer Alkohol/Wasser-Mischung in beliebigem Verhältnis löst, zu dieser Lösung eine wässrige Lösung von Natriumhydrogencarbonat in einem Verhältnis von 1 mol pro 1 mol Perindopril-Erbumin gibt und das Ganze mischt und durch Sprühen zu einer homogenen Mischung von inerten Bestandteilen zur Herstellung eines Granulats gibt, in einem Strom von warmer Luft mit einer Temperatur von mehr als 40°C trocknet, Substanzen zur Erleichterung der Tablettierung zugibt, homogenisiert und das Granulat formuliert.

2. Verfahren nach Anspruch 1, **dadurch gekennzeichnet, dass** man als inerte Bestandteile für die Herstellung des Granulats Lactose, Lactose in Monohydrat-Form, Trehalose, Maisstärke, mikrokristalline Cellulose, Talkum und Magnesiumstearat verwendet.

3. Stabile Formulierung von Granulat von amorphem Perindopril-Natriumsalz, die nach dem Verfahren der Ansprüche 1-2 erhältlich ist.

4. Formulierung nach Anspruch 3, **dadurch gekennzeichnet, dass** sie in Form eines Granulats, einer Tablette oder Kapsel vorliegt.

5. Verwendung der Formulierung nach den Ansprüchen 3-4 zur Herstellung einer Medizin mit blutdrucksenkender und gefäßerweiternder Wirkung.

## Revendications

1. Procédé pour la préparation, spécialement pour la préparation industrielle, d'une formulation stable d'un sel de sodium de périndopril amorphe de formule I : 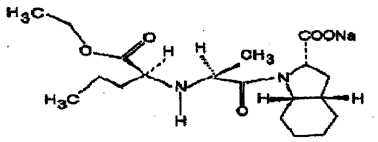 **caractérisé en ce que** du périndopril erbumine de formule II : 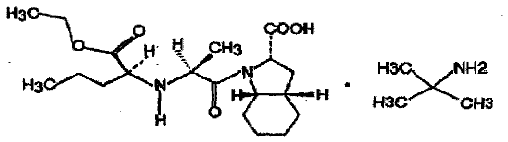 est dissous dans de l'eau ou dans un mélange alcool/eau en toutes proportions, on ajoute à cette solution une solution aqueuse d'hydrogénocarbonate de sodium dans un rapport d'une mole pour une mole de périndopril erbumine et le tout est mélangé et ajouté par pulvérisation à un mélange homogène d'ingrédients inertes pour la préparation d'un granulat, séché dans un courant d'air chaud dont la température dépasse 40
°C, on ajoute des substances pour faciliter le pastillage, on homogénéise et le granulat est formulé.

2. Procédé selon la revendication 1, **caractérisé en ce qu'**on utilise, comme ingrédients inertes pour la a préparation du granulat, du lactose, du lactose sous forme de monohydrate, du tréhalose, de l'amidon de maïs, de la cellulose monocristalline, du talc et du stéarate de magnésium.

3. Formulation stable de granulat de sel de sodium de périndopril amorphe obtenue selon le procédé décrit dans les revendications 1 et 2.

4. Formulation selon la revendication 3, **caractérisée en ce qu'**elle est sous la forme d'un granulat, d'un comprimé ou d'une capsule.

5. Utilisation de la formulation selon les revendications 3 et 4 pour la préparation d'un médicament possédant une action anti-hypertensive et vasodilatatrice.
